# EUROPEAN PATENT SPECIFICATION

(11) **EP 1 169 641 B1**
(45) Date of publication and mention of the grant of the patent: **17.10.2007**
(21) Application number: 00922084.9
(22) Date of filing: 12.04.2000
(51) Int. Cl.: G01N 33/53, G01N 33/50, G01N 33/68

(54) **PROTEOME MINING**
PROTEOMABBAU
CRIBLAGE A HAUT RENDEMENT DU PROTEOME

(30) Priority: 15.04.1999 US 129417 P; 05.05.1999 US 132595 P
(43) Date of publication of application: 09.01.2002
(73) Proprietor: THE UNIVERSITY OF VIRGINIA PATENT FOUNDATION, Charlottesville, VA 22903 (US)
(72) Inventor: HAYSTEAD, Timothy, A., J., Charlottesville, VA 22903 (US)
(74) Representative: Lucas, Brian Ronald
(86) International application number: PCT/US2000/009714
(87) International publication number: WO 2000/063694

(56) References cited:
- HAYSTEAD C M M ET AL: "GAMMA-PHOSPHATE-LINKED ATP-SEPHAROSE FOR THE AFFINITY PURIFICATION OF PROTEIN KINASES RAPID PURIFICATION TO HOMOGENEITY OF SKELETAL MUSCLE MITOGEN-ACTIVATED PROTEIN KINASE KINASE" EUROPEAN JOURNAL OF BIOCHEMISTRY, BERLIN, DE, vol. 214, no. 2, February 1993 (1993-02), pages 459-467, XP001156617 ISSN: 0014-2956
- BLUM ET. AL.: 'Isolation of Peptide Aptamers that Inhibit Intracellular Processes' PROCEEDING NATIONAL ACADEMY OF SCIENCE vol. 97, no. 5, February 2000, pages 2241 - 2246, XP002929894
- HANDFILED ET. AL.: 'Strategies for Isolation of in Vivo Expressed Genes from Bacteria' FEMS MICROBIOLOGY REVIEWS vol. 23, no. 1, January 1999, pages 69 - 91, XP002929895
- DAMER ET. AL.: 'Rapid Identification of Protein Phosphate 1-binding Protein by Mixed Peptide Sequencing Data Base Searching' THE JOURNAL OF BIOLOGICAL CHEMISTRY vol. 273, no. 38, 18 September 1998, pages 24396 - 24405, XP002929896

## Description

### US Government Rights

This invention was made with United States Government support under Grant No. DK52378A, awarded by the National Institutes of Health. The United States Government has certain rights in the invention.

### Field of the Invention

The present invention is directed to high throughput screening of proteomics for identifying bioactive compounds. More particularly the invention is directed to the isolation of novel herbicides, antibiotics, antifungals, antivirals, insecticide or pharmaceuticals based on their interactions with target molecules.

### Background of the Invention

The animal, plant, prokaryotic and viral kingdoms contain within them a vast array of genes that express 100,000's of distinct proteins whose biological function is essential life. The number of genes contained with in a particular organism varies greatly. Generally, the simpler the organism the fewer the total number genes. For example, completion of the yeast genome shows that these organisms have about 8300 genes, the complete *C*. *elegans* genome contains about 18,000 genes, and the human genome is estimated to contain about 100,000 distinct genes. Each gene encodes a specific protein which has a predetermined essential function for the over all survival of the organism. Collectively, given the biodiversity that exists on earth, the numbers of distinct genes that exist in nature is likely to number in the billions.

Obviously not all of the proteins expressed by the genes of an organism are likely to be of importance to man. Indeed the number of genes that are likely to express proteins of commercial or medical value is a tiny fraction of this vast biodiverse gene pool. Methods therefore that allow one to rapidly and effectively screen large numbers of proteins within this pool for valuable proteins are of great importance. In the case of the human genome it has been estimated that approximately 4000 of its genes are responsible for the causes of non-pathogen induced human disease. In short this means that human tissue contain 4000 proteins of potential medical and commercial value. The same analogies can be made to other species. For example if one was screening for a new antibacterial agent, one would be looking for bacterial protein targets that were peculiar to the particular bacterial strain of interest. In the case of bacteria this has traditional been enzymes involved in the synthesis of the bacterial cell wall. The classic example of a drug that is selective for bacteria is penicillin which inhibits an essential enzyme required for synthesis of the bacterial cell wall. Humans do not possess any of the enzymes that make bacterial cell walls. One cannot simply target any bacterial protein when searching for new antibiotics, this is because even though there are many differences between humans and bacteria, a significant portion of the bacterial genome encodes proteins of similar structure or function as found in humans. Drugs that inhibited proteins with a common function in both organisms are unlikely to discriminate between the two species.

To identify new drugs or commercially important bio-active molecules one needs methods that have the ability to encompass entire species genomes and immediately identify candidate proteins of importance. The present invention is directed to a method of identifying compounds that selectively interact with important biological components. This selective interaction is an essential element that makes a particular chemical have medical or commercial value. Without selectivity a compound has no bio-active value; selectivity is the single most important factor in all drug, antibiotic, antifungal, antiviral, insecticide and herbicide action.

The selectivity of a valuable bio-active compound in 99% of all cases is based on its interactions with one or more specific proteins contained within the target cell or organism of interest. One or two percent of valuable bio-active molecules maybe directed towards non-protein targets such as DNA, RNA, lipids or sugars. Without exception a valuable bio-active chemical interacts with its protein target in a highly specific manner. The target protein will contain on its surface a domain or pocket that binds the chemical with high affinity. This domain or pocket is unique to the target and not the several 1000 proteins that may also be contained with in the cell expressing the target protein.

In most cases the binding site for the chemical on the target protein is important to the biological activity of the protein. The binding site may be required for enzymatic activity, or be site of hormone interaction (e.g. a receptor) or a binding site for an all osteric regulator. When a chemical binds to one of these sites and affects the biological activity of the target protein it invariably contains structural components that resemble the natural biological ligand that normally occupies the site. The affinity of the interaction of the chemical for the target protein generally increases as the overall structural components of the chemical mimic the natural ligand. In some cases bio-active molecules fit better into the natural ligand-binding site than the natural ligand itself. These types of molecules are likely to have extremely potent bio-activity. If these molecules possess structural features that prevent their metabolism then this increases their bio-activity even greater.

One of the primary mechanisms for identifying bio-active chemicals of medical or commercial value is to screen large combinatorial libraries with some form of an enzymatic or biological assay. Combinatorial libraries can be extremely diverse and contain many hundred thousands of distinct molecules of known or unknown structure. They can be derived from very diverse sources, including plant extracts, animal extracts, soil samples, bacteria, fungi, chemical industry byproducts etc. Theoretically, these libraries contain within them molecules of every conceivable shape and form. However, like the proteins to be targeted, only a small percentage of these libraries contain molecules that have important bio-activity.

Prior high through put screens for drug discovery begin with a disease, the choice of which is invariably determined by potential market size. The etiology of the disease is first defined by basic research to determine likely underlying cause. This research identifies potential protein targets that may be useful drug targets; e.g. receptors or enzymes. The purified receptors or enzymes are then used to screen chemical libraries for agents either bind, inhibit or activate. Similar approaches are also used to screen for anticancer drugs. Transformed cell lines are used to screen large chemical libraries that may contain compounds that revert them to their normal phenotype or kill the cancer. Further investigation is then used to identify the molecular mechanisms by which active compounds from these screens bring about their cellular effect.

Therefore in the traditional search for a new bio-active compounds one begins with a specific biological problem in mind. For example a particular pharmaceutical house maybe focused on discovering new antihypertensive agents. Its decision to enter into the screening process is always based on the disease market size. Thus the search for drugs that would treat small populations of afflicted individuals is unlikely to happen in the private sector. In the case of a new antihypertensive agent for example, a drug screen will generally begin with an assay that includes a specific receptor or enzyme that has important functions in the regulation of blood pressure. One then has to hope that the libraries one screens contain bio-active molecules that selectively effect the proteins selected in the assay. Once candidate chemicals are identified one then has to demonstrate that these compound act selectively and predictably for the targeted protein in the assay and not others. Thus in the initial stages one ends up with many false positives which must be eliminated in a second round of screening because the entire expressed genome was not taken into account in the first instance. The invention described herein eliminates this problem at the start because it encompasses both the diversity contained within the chemical library to be screened, with the diversity of the expressed genome itself in one step.

The selectivity is achieved in the analysis following sequencing of the targeted proteins. A decision as to whether a particular protein/chemical interaction is likely to have commercial and medical value is made during the last stages of analysis. Therefore, in addition to identifying bio-active agents that have commercial value the screen of the present invention does not exclude compounds that may have humanitarian value. This is because we could conceivably identify agents that bind to proteins important in the pathology of obscure diseases with small patient populations.

Finally, the present invention can readily cross platforms with no change in protocol or equipment. There is no difference in screening procedures for herbicides, antibiotics, antifungals, antivirals, insecticide or pharmaceuticals. All one changes is the expressed genome (proteome) that is to be screened. One can even use the same libraries for each screen; i.e. a library that did not yield any useful pharmaceutical agents may contain a useful herbicide.

### Summary of the Invention

The present invention is directed to compositions and methods for identifying bioactive compounds. Advantageously, the present method of identifying bioactive compounds utilizes both the diversity contained within a chemical library to be screened, with the diversity of the expressed genome itself in one step to maximize the efficacy of the screening procedure.

The invention provides a method of identifying target compounds bioactive towards one or more proteins present in a proteome, which comprises:
providing a solid support on which a ligand is immobilised;
contacting the solid support with protein members of a proteome to bind some of said protein members to said ligand on said support;
washing the support to remove non-specifically bound proteins;
contacting the support with one or more target compounds that compete with said immobilised ligand for a binding site on one or more of said protein members of said proteome to release one or more bound proteome proteins;
collecting the protein or proteins released from said support by said one or more target compounds; and
determining for the target compound or compounds by sequencing or mass spectroscopy and comparison to protein databases the identity of the protein or proteins released from the support for identification of target compounds and/or proteins.

### Brief Description of the Drawings

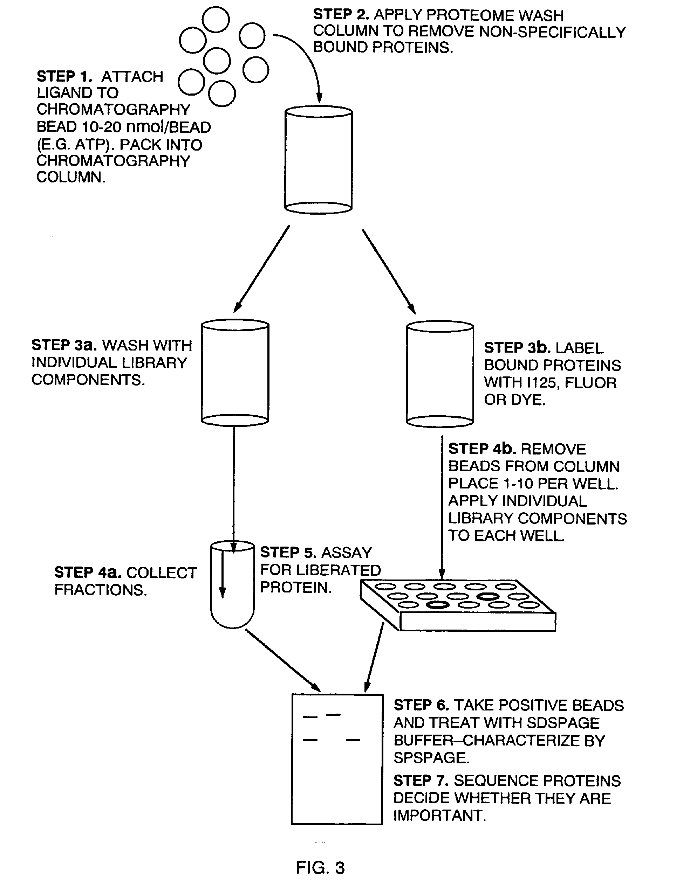

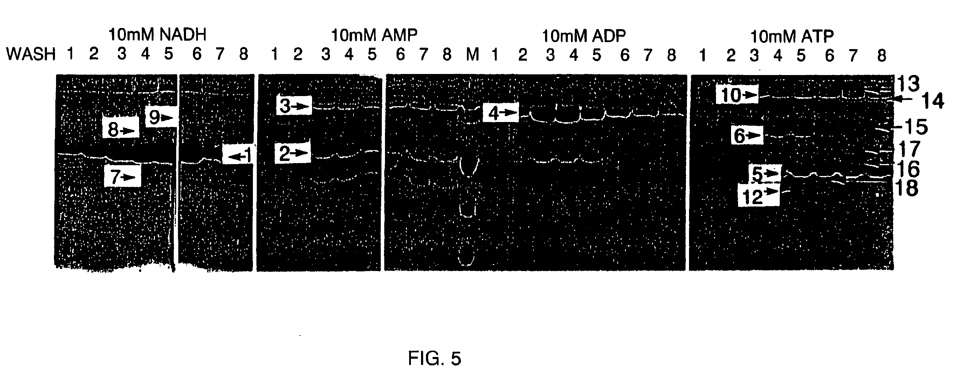

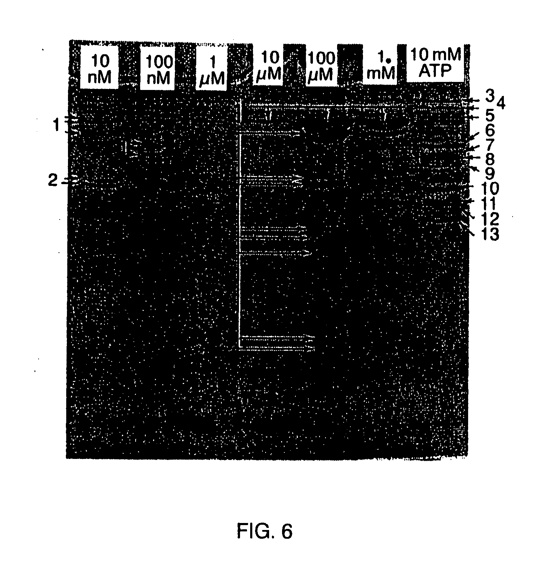

Fig. 1 is a diagramatic representation of the steps used in accordance with one embodiment to isolate bioactive compounds from a complex mixture of proteins (proteome) using an immobilized ligand.

Fig. 2 represents the stained SDSPAGE results from characterization of proteins isolated from rabbit skeletal muscle through the use of gammaphosphate linked ATP-Sepharose. Rabbit skeletal muscle extract was prepared from 350 g of tissue (w/w) and passed over 50 mls of gamma phosphate linked ATP-Sepharose containing 10 umols/ml of linked ATP. Following washing, the column was eluted sequentially with NADH, AMP, ADP and ATP and fractions collected (10mls). Column fractions were separated by SDSPAGE then transfered to PVM and stained with amido black. Proteins 1-17 were identified by mixed peptide sequencing (see Table 1).

Fig. 3 represents the stained SDSPAGE results from geldanamycin released muscle extract proteins. ATP-Sepharose was loaded with skeletal muscle extract and eluted successively with the indicated concentrations of geldanamycin, followed by 10 mM ATP. Peak fractions (20ul of 1.0) were analyzed by SDSPAGE and silver staining or transferred to PVM for identification by peptide sequencing. Numbers indicate that proteins that wee identified on the PVM membrane: 1. HSP90 and proteolytic fragments of HSP90; 2. purine synthetase (ADE2); 3. myosin light chain kinase; 4. phosphorylase kinase; 5. p98 glucose indued kinase; 6. HSP70; arginine succinate synthetase; 7. glutamate dehydrogenase; 8. glutamate ammonium ligase; 9. glutathione synthetase; 10. aldehyde dehydrogenase; 11. MAPK; 12. GAPDH; 13. PKA

### Detailed Description of the Invention

In describing and claiming the invention, the following terminology will be used in accordance with the definitions set forth below.

As used herein, "nucleic acid," "DNA," and similar terms also include nucleic acid analogs, i.e. analogs having other than a phosphodiester backbone. For example, the so-called "peptide nucleic acids," which are known in the art and have peptide bonds instead of phosphodiester bonds in the backbone, are considered within the scope of the present invention.

As used herein, bioactive compounds include any compound that is capable of inducing an effect on a living cell or organism. Bioactive compounds include but are not limited to pharmaceuticals, hormones, chemotherapeutics, nucleic acids and the like.

As used herein the term "proteome" relates to a complex mixture of proteins that are derived from a common source, such as an extract isolated from a particular cell or tissue. For example a human proteome represents a mixture of proteins isolated from human cells. The category can be further defined by specifying a particular cell/tissue source for the proteome (i.e. a human myocardial tissue proteome represents all the proteins isolated from human myocardial tissue).

As used herein the term "combinatorial library" relates to a collection of compounds. The combinatorial library can be a biological synthesized library that comprises nucleic acid sequences that include a common vector sequence (allowing for replication of the library in a host species) and a protein encoding region. The biological synthesized library can be further provided with regulatory elements that allow for the expression of the encoded proteins (i.e. an expression library). Chemical libraries are collections of compounds that were isolated from a natural source or were synthesized in a laboratory using chemical or biological processes. A "combinatorial chemical library" is a collection of compounds created by a combinatorial chemical process, wherein the compounds of the combinatorial chemical library have a common scaffold with one or more variable substituents.

As used herein the term "solid support" relates to a solvent insoluble substrate that is capable of forming linkages (preferably covalent bonds) with soluble molecules. The support can be either biological in nature, such as, without limitation, a cell or bacteriophage particle, or synthetic, such as, without limitation, an acrylamide derivative, agarose, cellulose, nylon, silica, or magnetized particles.

As used herein the term "naturally-occurring" relates compounds normally found in nature. Although a chemical entity may be naturally occurring in general, it need not be made or derived from natural sources in any specific instance.

As used herein the term "non naturally-occurring" relates to compounds rarely or never found in nature and/or made using organic synthetic methods.

As used herein the term "functional analog" of a library compound/ligand relates to a compound that has a binding affinity for the same ligand as one of the members of the library, such that the functional analog will compete with the library component for binding to that ligand.

The present invention is directed to a novel method for the rapid identification of bioactive compounds, including but not limited to novel drugs, antibiotics, antifungals, antiviral, insecticide or herbicides. The overall strategy behind the invention is to screen complex protein mixtures with a library of compounds for proteins that bind specifically to components in the library. The bound proteins are then identified by protein microsequencing to determine if the identified protein is therapeutically relevant. The protein has therapeutic relevance if the protein is known to be central to the development of a disease, is a metabolic enzyme unique to a particular microorganism, yeast, virus, or fungi, or is an enzyme peculiar to a type of insect, or an enzyme required for photosynthesis in a particular weed.

The advantage of the present screening methodology derives from the initial assumption that the entire genome is a potential drug target. The only decision prior to screening is to decide what proteome should be utilized; i.e. for drugs important in human disease, human tissue is the choice, for herbicide a plant species, for antibiotic, a bacterial strain.

In one aspect of the invention, systems and methods are provided for rapidly screening a combinatorial library for bioactive agents. The method is based on the identification of those library components that interact with proteins of a preselected proteome, wherein the proteome protein is a potential target for therapeutics. The method of identifying bioactive compounds comprises the steps of contacting a combinatorial library with the protein members of a proteome under conditions that allow for specific interactions between proteins of the proteome and the library. Proteins that interact with the library components are then isolated and analyzed to determine if the protein is interesting from a therapeutic standpoint. Those proteins that have therapeutic relevance are then used to identify the component of the library that interacts with the protein.

In one embodiment, the method of identifying bioactive compounds present in a compound library comprises the steps of contacting members of a compound library with the protein members of a preselected proteome bound to an immobilized ligand. Bound proteins may be released from the solid support by contacting the bound proteins with one or more individual members of the compound library or with functional analogs of the library components. The released proteins are then identified by protein sequencing or mass spectrometry; and the identity of the specific compounds of the compound library that bind to the released proteins is determined.

### The libraries

Combinatorial libraries can be constructed using techniques known to the skilled practitioner to provides researchers vast number of chemical candidates to screen for potential bioactivity. In accordance with the present invention the library comprises a collection of compounds that are capable of specific binding to their target. For example, suitable library components include, but are not limited to peptides, proteins, carbohydrates, lipids, glycoproteins or nucleic acids.

Biologically synthesized combinatorial libraries have been constructed using techniques of molecular biology. These library components are expressed using bacteria or bacteriophage particles. For example, U.S. Pat. No. 5,270,170 and 5,338,665 to Schatz describe the construction of a recombinant plasmid encoding a fusion protein created through the use of random oligonucleotides inserted into a cloning site of the plasmid. In other biological systems, for example as described in Goedell et al., U.S. Pat. No. 5,223,408, nucleic acid vectors are used wherein a random oligonucleotide is fused to a portion of a gene encoding the transmembrane portion of an integral protein. Upon expression of the fusion protein it is embedded in the outer cell membrane with the random polypeptide portion of the protein facing outward. Thus, in this sort of combinatorial library the compound to be tested is linked to a solid support, i.e., the cell itself and the cell itself adheres to the cell culture substrate. The Goedell patent is incorporated herein by reference.

Similarly, bacteriophage display libraries have been constructed through cloning random oligonucleotides within a portion of a gene encoding one or more of the phage coat proteins. Upon assembly of the phage particles, the random polypeptides also face outward for screening. Such phage expression libraries are described in, for example, Sawyer et al., 4 Protein Engineering 947-53 (1991); Akamatsu et al., 151 J. Immunol. 4651-59 (1993), and Dower et al., U.S. Pat. No. 5,427,908. These patents and publications are incorporated herein by reference.

While synthesis of combinatorial libraries in living cells has distinct advantages, including the linkage of the compound to be tested with its nucleic acid, there are clear disadvantages to using such systems as well. The diversity of a combinatorial library is limited by the number and nature of the building blocks used to construct it; thus modified or R-amino acids or atypical nucleotides may not be able to be used by living cells (or by bacteriophage or virus particles) to synthesize novel peptides and oligonucleotides. There is also a limiting selective process at play in such systems, since compounds having lethal or deleterious activities on the host cell or on bacteriophage infectivity or assembly processes will not be present or may be negatively selected for in the library.

Another approach to creating molecularly diverse combinatorial libraries employs chemical synthetic methods to make use of atypical or non-biological building blocks in the assembly of the compounds to be tested. Thus, Zuckermann et al., 37 J. Med. Chem. 2678-85 (1994), describe the construction of a library using a variety of N-(substituted) glycines for the synthesis of peptide-like compounds termed "peptoids". The substitutions were chosen to provide a series of aromatic substitutions, a series of hydroxylated side substitutions, and a diverse set of substitutions including branched, amino, and heterocyclic structures. This publication is incorporated by reference herein.

Alternatively, chemical synthetic methodologies can be used to create large diverse libraries of potentially useful compounds and permits the synthesis of compounds joined to a solid support of some kind or joined to an identifiable marker such as a flourescent tag. In accordance with one embodiment, the combinatorial library is chemically synthesized on solid supports in a methodical and predetermined fashion, so that the placement of each library member gives information concerning the synthetic structure of that compound. Examples of such methods are described, for example, in Geysen, U.S. Pat. No. 4,833,092, in which compounds are synthesized on functionalized polyethylene pins designed to fit a 96 well microtiter dish so that the position of the pin gives the researcher information as to the compound's structure. Similarly Hudson et al., PCT Publication No. WO94/05394, describe methods for the construction of combinatorial libraries of biopolymers, such as polypeptides, oligonucleotides and oligosaccharides, on a spatially addressable solid phase plate coated with a functionalized polymer film. In this system the compounds are synthesized and screened directly on the plate. Knowledge of the position of a given compound on the plate yields information concerning the nature and order of building blocks comprising the compound.

Another approach has been the use of large numbers of very small derivatized beads, which are divided into as many equal portions as there are different building blocks. In the first step of the synthesis, each of these portions is reacted with a different building block. The beads are then thoroughly mixed and again divided into the same number of equal portions. In the second step of the synthesis each portion, now theoretically containing equal amounts of each building block linked to a bead, is reacted with a different building block. The beads are again mixed and separated, and the process is repeated as desired to yield a large number of different compounds, with each bead containing only one type of compound. This methodology, termed the "one-bead one-compound" method, yields a mixture of beads with each bead potentially bearing a different compound. The compounds displayed in the surface of each bead can be tested for the ability to bind with a specific compound (i.e. a protein member of a proteome).

The libraries used in the present invention can be well defined, containing known mixtures of molecules, or the library can be one in which the chemical content is poorly defined.

In cases where a target compound is identified that interacts with a large number of proteins (e.g. ATP, see Fig 1) only the target compound is immobilized on the solid support. The bound proteins can be released by the addition of an excess of free target compound, a derivative of the target compound or a functional analog of the target compound (see Example 2).

### The proteomes

The libraries are contacted with a proteome under conditions conducive to the formation of specific interactions between components of the proteome and components of the library. Choice of the proteome used depends on the problem being addressed. If one is looking to isolate a new drug for the treatment of a human disease the human proteome (i.e. human tissue) is the best choice. If one wishes to discover a new antibiotic then the target pathogen (e.g. a gram negative bacterium) of interest is the obvious choice. For an insecticide, the targeted insect and so on.

In accordance with one embodiment the proteome comprises a natural products library which represents a collection of natural products that have been recovered from biological material and have been determined to have biological activity. For example the natural products library may include a mixture of natural products wherein the mixture is known to induce a phenotypic change in a population of cultured cells.

The amount of starting tissue used to isolated the proteome proteins is critical and should be based on theoretical recovery of target proteins. For example, if one is interested in identifying drugs that may bind to signal transduction molecules one should take into account the amount of these proteins (or copy number) that may be in the cell. Many of these types of molecules may be expressed as low as 200 copies per cell. A quick calculation predicts that if one wanted to recover as much as 10 pmol of a particular protein that was expressed at 200 copies per cell one would require 12 grams of wet weight tissue. For high copy number proteins, such as metabolic enzymes, less tissue mass would be required to achieve 10 pmol of protein. One should also factor in potential losses due to inefficiency of extraction or proteolysis. Thus although 12 g of tissue may contain a total of 10 pmol of a target protein of interest one may only recover a small percentage of this in the initial screen. Fortunately, modem protein detection and sequencing methods enable one to identify proteins in the femto molar range. However, increasing the starting tissue mass as much as possible will improve the odds of recovering sufficient protein for later identification.

Tissue from the chosen target proteome is ground and homogenized in buffers appropriate for solubilizing proteins and retaining their native conformations. This involves standard procedures utilized in most biochemical laboratories. Following clarification by centrifugation the portions of the extraction mixture are passed over the immobilized ligand.

In accordance with one embodiment certain components of the proteome can be removed prior to contacting the immobilized ligand with the proteome. Components can be removed, for example, by fractionating the components based on molecular weight, electric charge and/or hydrophobicity. Alternatively, specific components can also be removed by ligand or antibody binding. Such methods allow the removal of protein components that do not warrant further investigation but are know to bind to certain components of the target compound library. In addition such prescreening allows for the removal or reduction of proteins that are expressed at high levels in the tissue used to generate the proteome.

The compounds of the proteome are placed in contact with the library component under conditions favorable for specific interactions between members of the two groups. The interaction may result in the alteration of a physical characteristic such as fluorescence, absorption, enzymatic activity, but typically the specific interaction involves binding of the two components to one another.

### Isolation and identification of target proteins with a defined library

As outlined in Fig. 1 a ligand that interacts with many protein targets can be used to identify important drugs. In accordance with this embodiment a proteome is passed over a resin containing a single ligand (e.g. gamma-phosphate linked ATP Sepharose). Following washing to remove non-specifically bound proteins, either the bound proteins are labeled as described below or the column is successively washed with components in a chemical library and fractions collected as described below. If the proteins are labeled then the beads are removed from the column and placed into microtitre plate wells (1-10 beads/well to give ~20 nmols total protein). Individual components in the library are then applied to each well and the supernatants sampled for protein release. Preferably, each library component should be added at increasing doses in orders of magnitude ranging from 1 nM to 1 mM. Proteins that are selectively liberated in the nm-µM range are analyzed by SDSPAGE and mixed peptide sequencing is conducted as described below.

In the case of resins packed in chromatography columns, each component in the library is passed separately over the immobilized proteome and any eluted material collected. It is anticipated that a certain portion of the library components will be able to compete with the bound ligand and selectively liberate one or more proteins from the resins in the plates or columns. A portion of each eluate is then placed into a microtitre plate for protein analysis. Advantageously, using this method identifies the specific library component that binds to the released protein. Further analysis of the protein will determine if this binding interaction has potential therapeutic value.

If the column is broken down and distributed into the individual wells of a microtitre plate prior to releasing the bound proteome proteins, an additional step must be taken to isolate the released proteins. Library components are added to each well and incubated to release the bound proteins. Following incubation with each library component, the resin is allowed to settle or the resin suspension is centrifuged briefly (300 x g) to pellet the beeds. In accordance with one embodiment the resin beads can be magnetized beads, and a magnetic field is applied to the bottom of the plate to hold the resin at the bottom. After the resin has been separated from the supernatant, a portion of the supernatant from each well is removed and placed in a second well containing a high sensitivity protein staining reagent. This last step can be automated using standard robots familiar to those skilled in the art.

The protein detection reagent used to detect the presence of released protein can be any of those known to the skilled practitioner. In one preferred embodiment the detection reagent is one that changes color in the presence of protein. Radioactive isotopes that bind proteins (I¹²⁵), fluorophors (e.g. FITC) or gold stains may also be used to increase sensitivity. Specialized detection systems capable of detecting these markers are known to those skilled in the art.

Wells/column fractions that are positive for protein are selected for further analysis using standard techniques. It is anticipated that the number of positive wells would be a few percent of the total number of components that are in the library screened - although this may be several 100 if the total number of components in the initial library numbers in the 10,000's. The proteins will be subjected to gel electrophoresis analysis, typically using SDSPAGE, to measure the purity of the protein and quantitate the amount of recovered protein. The supernatant from each well that contains liberated protein(s) is mixed with SDSPAGE sample buffer and characterized by SDS gel electrophoresis. Many hundreds of supernatants can be easily characterized using this method. The gel is stained with silver, Commassie or colloidal gold (for sequencing by mass spectrometry) or transferred to polyvinyl membrane (for mixed peptide sequencing). At this point the molecular weights and amount of protein recovered are determined.

For mixed peptide sequencing, the proteins on the polyvinyl membrane (PVM) are stained then excised (See Damer et al., (1998) J. Biol. Chem 273: 24396-24405). The membrane pieces are digested briefly with CnBr, washed and placed directly into an automated Edman sequenator. Mass spectrometry can also be used but may be less desirable because of the amount of labor that is required and its inability to handle many protein samples at one time. In the case of mixed peptide sequencing between 6 and 12 rounds of Edman sequencing are carried out and the mixed peptide sequences generated sorted and matched against the databases with the FASTF (protein databases) and TFASTF algorithms (DNA data bases). This process identifies the liberated proteins in each well.

At this point a determination is made as to whether or not the liberated protein is interesting (i.e. is the protein involved in a human disease, is it an important enzyme to bacterial metabolism.. etc). If the protein is determined to have therapeutic value, then the chemical that liberated the protein from the immobilized ligand is chosen for further characterization using conventional approaches. For example, an affinity assay will typically be conducted to determine if the protein has sufficient affinity for the target library compound (i.e. binding at nanomolar concentrations) to be useful as therapeutic agent. In addition, analysis will be conducted to determine what is the biological impact of the interaction and whether the affinity of interaction with the targeted protein can be improved by modification.

This screen may yield several candidate proteins that are valuable in the initial round and not confine one to a single field or market or interest. Importantly, a single round of screening will not only identify a potentially useful bioactive agent, but will also provide valuable information about its targets, what groups can be modified without affecting function and where the agents should be applied.

In accordance with one embodiment the method of identifying bioactive compounds from a complex mixture of proteins comprises contacting a combinatorial library with the complex mixture of proteins under conditions that allow for specific binding of the proteins to library components.

The proteins bound to library components by specific interactions are then released, preferably by a competition reaction using one or more of the components of the library (in an unbound state). Wherein the library has been fractionated and equal portions of the support particles have been distributed into a plurality of wells of a microtitre plate the step of releasing the bound proteins comprises adding to each microtitre plate well one or more compounds of the library. The released proteins are characterized using standard techniques and the compounds of the library that specifically bind to the released proteins are identified.

The released proteins will be identified primarily based on microsequence analysis and comparison to existing protein databases. This has been made feasible because of the near completion of the nucleotide sequencing of several genomes, including human, mammalian, C.elegans, bacteria, yeast, viral, rice, corn. The invention will have increasing relevance as more species specific genomes become complete.

### Isolation and identification of target proteins with an undefined chemical library.

All of the initial steps are the same as described above. Following preparation of the resins and application of protein mixture, the resins are again aliquoted into titre plate wells. However, since the components in the library are poorly defined and of unknown number some fractionation of the library using standard methods is required. Useful steps for fractionation include organic and aqueous extraction, HPLC or ionic-exchange fractionation. The fractionated library is then applied to each well containing resin and incubated as described. In one embodiment these steps are carried out robotically. The column chromatography approach outlined above is also applicable with an undefined library.

Following incubation with each fraction of the library the resin is pelleted as described above and a sample of the supernatant taken for protein analysis. Fractions that contain liberated protein are selected for characterization by SDSPAGE. At this point it is likely that some fractions of the library will liberate many proteins, some only a few. In either case, mixed peptide sequencing or mass spectrometry can be used to identify all these components in a short space of time. With mixed peptide sequencing a standard Edman sequencer containing 4 reaction chambers can identify 20-30 proteins per week. Mass spectrometry will be somewhat slower if a species-specific database is not available. The list of proteins that are identified for each well is then surveyed for the criteria stated earlier.

Proteins that are deemed valuable are expressed as recombinant proteins and immobilized on a second resin (Sepharose or agarose beads). The fraction of interest or entire chemical library is then passed over the protein affinity column to selectively recover the chemical compounds with in the library that bind the protein of interest. Mass spectrometry or NMR techniques can then be used to identify or determine the structure of the bioactive compound. One then proceeds with the standard methods to characterize the bioactivity of the isolated chemical. The strategy as outlined in Fig. 1 can be applied to an undefined library.

### Example 1

### Isolation of Adenine Nucleotide Binding Protein from a Proteome

As a proof of principle and to evaluate the types of proteins that bind to gamma-phosphate linked ATP-Sepharose, tissue extracts prepared from rabbit skeletal muscle, liver, kidney, brain or bladder were passed over a gamma-phosphate linked ATP-Sepharose affinity resin. Following extensive washing to remove non-specifically associated proteins, the resin was washed sequential with NADH, AMP, ADP and ATP. Fig. 2 shows the results from characterization of proteins isolated from skeletal muscle. Similar results were obtained with other tissues, although the pattern, abundance and complexity between tissues varied considerably due to varied levels of expression of individual proteins (See Fig. 2 and Table 1).

Gamma phosphate linked ATP-Sepharose was washed with extracts prepared from rabbit, skeletal muscle, kidney, liver, brain or bladder. Following washing the resin was eluted successively with the indicated nucleotides as described in Fig. 2.

Proteins 1-17 (see Fig. 2) were identified by mixed peptide sequencing (Table 1). Eluted proteins were characterized by SDSPAGE, transferred to PVM and treated with CnBr or Skatol prior to mixed peptide sequencing. Mixed peptide sequencing was carried out on average for 6-12 Edman cycles. The mixed sequences were sorted and matched against the entire published protein or DNA data bases with the FASTF or TFASF algorithms respectively (Damer et al. 1998., Alms et al. 1999). Expectation scores for the identified proteins ranged from 2.6 e⁻⁷ for PKA to 1.2 e⁻⁵⁴ for GAPDH. Expectation scores after each search for the next highest scoring non-related protein were generally < 2.3 e⁻⁴ The experiment shown was repeated on several occasions, and on several different tissue including liver (120g), kidney (60g), brain (60g) and bladder (20g) with similar results (Table 1).

**Table 1**

| NADH | pmol | AMP | pmol |
|---|---|---|---|
| GAPDH (M) 1* | 25,000.0 | Purine synthetase (M,L) 2* | 5.0 |
| malate dehydrogenase (M) 8* | 0.5 | Phosphorylase (M) 3* | 10.0 |
| glutamate dehydrogenase (L) 9* | 1.5 | AMP activated protein kinase (L) # | 2.0 |
| aldehyde dehydrogenase (M) 7* | 2.0 | EST AA254816 (L) | 4.0 |
| lactate dehydrogenase (M,L) # | 50.0 | ESTAA571903 (L) | 5.0 |
| 6-phosphogluconate dehydrogenase (M,L) | 5.0 | phosphatidylinositol-4-phosphate 5-kinase (L) | 2.0 |
| isocitrate dehydrogenase (L) | 1.0 | protein kinase DUN 1 (related) (L) | 1.0 |
| 3-hydroxyacyl-CoA dehydrogenase (L) | 1.0 | | |
| sorbitol dehydrogenase (L) | 2.0 | | |
| alcohol dehydrogenase (M,L) | 20.0 | | |
| glucose-6-phosphate dehydrogenase (M,L) | 17.0 | | |

| ADP | pmol | ATP | pmol |
|---|---|---|---|
| Heat shock protein 90 (M/L) 4*# | 15,000.0 | MAPK (M) 5*# | 5.0 |
| Purine synthetase (M/L) 2* | 14.0 | MEK 1 (M)# | 6.0 |
| | | Pyridoxal kinase (M, L) 12* | 10.0 |
| | | Arginnosuccinate synthetase (M) 11 * | 1.0 |
| | | Glutamate ammonium ligase (M,L) 6* | 2.0 |
| | | Adenosine kinase (M,L) 15* | 5.0 |
| | | CSK (M) 16*# | 4.0 |
| | | HSPA5(M)17* | 4.0 |
| | | P90 S6 kinase (M, L) 14* | 0.5 |
| | | P70 S6 kinase (L) | 0.2 |
| | | Pyridoxal kinase (L) | 5.0 |
| | | P98 glucose induced kinase (M,L,SM,B,K) 10* | 10.0 |
| | | Heat shock protein 70 (L,M,B,SM,K) | 55.0 |
| | | PKA (L,M,B,SM) | 6.0 |
| | | Glutamine synthetase (L) | 9.0 |
| | | ZIP Kinase (SM)# | 0.2 |
| | | | |
| | | Phosphofructokinase (L) | 6.0 |
| | | Heat shock protein 60 (M,L,B,SM,K) | 10.0 |
| | | RNA helicase (L) | 1.0 |
| | | Protein kinase PC-1 (L) | 0.6 |
| | | Protein kinase C epislon (L)# | 0.2 |
| | | Beta tubulin (B)# | 50 |
| | | P6 electron transport flavoprotein α subunit (L) | 1.0 |
| | | serine/theonine-protein kinase ip11 (related) (L) | 0.2 |
| | | protein kinase C beta-II (L) | 0.2 |
| | | protein kinase kem (L) | 0.3 |
| | | cyclic G kinase (SM)# | 5.0 |
| | | lupus nephritis protein LN1 (SM) | 1.0 |
| | | casein kinase 1 (M) | 5.0 |
| | | casein kinase 11 (M) | 6.0 |
| | | GSKIII (M) | 4.0 |
| | | lim domain kinase 1 (SM) | 0.2 |
| | | protein kinase pkx1 (SM) | 0.3 |
| | | pp60c-src (M) | 1.0 |
| | | MLCK (M,SM)# | 6.0 |
| | | Phosphorylase kinase (M) | 20.0 |
| | | Arginine deimidase (M) 18* | 5.0 |
| | | CAM kinase II (B,M)# | 1.0 |
| | | fructose-1-6-bisphosphatase (M,L) | 2.0 |

| | | | |
|---|---|---|---|
| The pmol amount of protein recovered was determined from PTH amino acid recovery during mixed sequencing multiplied by the volume applied to the gel and fraction volume (mls). *Indicates proteins identified in Fig. 2; # indicates proteins tested for binding efficiency by Western analysis of the column flow through. | | | |

To unambiguously identify the eluted proteins in each case, peak column fractions were transferred to PVM following SDSPAGE and subjected to mixed peptide sequencing (Table 1). Table 1 shows the identification of over 70 proteins that were eluted from ATP Sepharose loaded with skeletal muscle, liver, brain, kidney or bladder. Without exception, all of the proteins identified in the protein data bases belonged either to the protein kinase, dehydrogenase or ATP-grasp classes of purine binding proteins. Analysis of PTH amino acid recovery during mixed peptide sequencing reveals that the affinity resin recovered proteins of both high and low cell copy number (Table 1). Western blotting of the column flow through with antibodies to several of the identified proteins demonstrated that the resin absorbed the tested proteins with >85 % efficiency from the initial extract (Table 1). This finding indicated that the differences in recovery of individual proteins in the nucleotide washes was a reflection of cell copy number rather than because of affinity differences for the immobilized ATP.

Several of the identified proteins have been crystallized with NADH, AMP, ADP or ATP bound and these published structures explain selective recovery of each classification of protein from the affinity resin. Inspection of the three dimensional structure of 10 of the dehydrogenases identified in the NAD wash shows in each case the adenine portion of these nucleotides is buried within a cleft containing the conserved Gly-X-Gly-X-X-Gly loop. The diphosphate portion of the bound nucleotides spans an open region on the surface connecting to the nicotinamide moiety accommodated within a closely situated second binding site.

The finding that 0.5 mM NADH/NAD exclusively eluted dehydrogenases over other purine binding proteins is consistent with the well established preferences of these types of enzymes for nicotinamide containing purines. Although it should be noted that in separate experiments increasing the concentration of NADH/NAD to >10 mM did begin to elute many of the proteins found in the subsequent AMP wash. Characterization by microsequencing of the AMP eluate identified two proteins that are allosterically regulated by the nucleotide, glycogen phosphorylase and the AMP activated protein kinase. In the case of phosphorylase, elution with AMP is consistent with the crystal structure of enzyme in its 'T' state, which is favored by the presence of glucose, ADP and ATP competitors, low concentrations of substrate (Sprang et al. 1991). Purification of the AMP activated protein kinase over gamma phosphate linked ATP-Sepharose has been reported previously (Davis et al 1996). The enzyme is known to contain both an ATP and AMP binding pocket and is activated by AMP *in vitro.* Recovery of the kinase with AMP therefore is most likely because of interaction with the immobilized ATP with its AMP binding pocket. Elution of multifunctional protein ADE2 with AMP (and also ADP) is consistent with involvement of this protein in catalyzing the conversion of AIR to CAIR (steps 6 and 7) in purine biosynthesis. Although the three dimensional structure of mammalian ADE2 has not been solved, the related *E.coli* enzyme, N5 - carboxyaminoimidazole ribonucleotide synthetase (PurK) with ADP bound was recently reported (Thoden et al. 1999). PurK belongs to the ATP grasp superfamily of purine binding proteins, and in prokaryotes, plants and yeast, catalyzes the conversion of AIR to CAIR in a two step process (steps 6 and 7 of 10) involving a second distinct gene product PurE. Recovery of mammalian ADE2 from the affinity resin in this present study suggests it also binds purine nucleotides in a similar orientation to that found in PurK. Phosphatidylinositol-4-phosphate 5-kinase, protein kinase DUN1 (related) and the two EST AA254816, ESTAA571903 all contain nucleotide binding motifs in their primary sequence and elution of these proteins with AMP suggests that they also bind the purine orientating the phosphate such that it is solvent accessible.

Elution of the resin with ADP following AMP eluted two proteins, HSP90 and ADE2 in all tissues tested. The recovery of additional ADE2 with ADP suggests that this enzyme may either have two separate adenine binding pockets or exist in two conformational states that discriminate the presence of _ and _ phosphates on the two types of purine. Recovery of HSP90 with ADP is consistent with recent reports by Toft and coworkers identifying the N terminal domain of HSP 90 as a Mg 2+ ATP/ADP binding domain and the crystal structure of this domain with ADP or ATP bound (Prodromou et al. 1997, Stebbins et al. 1997 35-38). Interestingly, the purine binding pocket on HSP90 was not readily predicted to exist based on primary sequence alignments alone. Recovery of HSP90 suggests that other non-conventional purine binding proteins presenting adenine containing nucleotides in the "protein kinase" orientation are likely to be recovered from the affinity resin. Examples of other proteins that been crystallized with purine bound and classified as having non-conventional binding domains are the adenine binding domain of DNA gyrase B (Wigleyet al. 1991), the AMP binding sites on glycogen phosphorylase and adenylate kinase, the ADP binding sites on fructose-1-6-bisphosphatase and phosphofructokinase, the cyclic AMP binding sites on catabolite activator protein and ATP binding site in DD-ligase. Notably four of these proteins were subsequently recovered in the ATP wash (Table 1).

Final elution of the affinity resin with ATP eluted a diverse range of proteins in all tissues tested, from heat shock proteins and metabolic enzymes with non-conventional nucleotide binding folds to a variety of protein kinase family members (Table 1). The majority of the proteins recovered are known to utilize Mg □□ ATP and show a high degree of specificity towards the nucleotide. Consistent with this observation, we have found that inclusion of mM NADH, AMP and ADP in the extraction buffer completely abolishes binding of all of the proteins shown in Table 1 that would normally be recovered from the resin in their absence (data not shown). In contrast, proteins identified in the ATP elution are retained on the resin under these conditions. Amongst the most frequent of all the adenine binding proteins sequenced in Table 1 are protein kinases. It has been estimated that up to 2% of the entire human genome may encode a protein kinase with a highly conserved ATP binding cassette. When the amino acid sequences of over 400 protein kinases are aligned with that of cyclic AMP dependent protein kinase, 15 amino acids residues within 11 conserved subdomains are nearly invariant. In addition, there are 19 hydrophobic amino acids of similar structure that are also conserved within the protein kinase family. In the activated state, the conserved and invariant amino acids of the ATP binding cassette make intimate contact with MgATP and orientate the molecule such that its gamma phosphate is exposed at the lips of the catalytic cleft. The recovery of several distinct tyrosine and serine/threonine protein kinases as reported herein, and reports by others utilizing gamma phosphate linked ATP-Sepharose in purification schemes for specific kinases, demonstrates that this affinity resin is likely to bind all protein kinase family members. Furthermore, this finding, combined with the frequency of occurrence of protein kinases, dehydrogenases and some of the other proteins identified in Table 1 in the current protein and DNA data bases suggests that the ATP resin may catch 3-5% of all proteins present in most eukaryotic genomes.

### Example 2

### Screening for Selective Inhibitors for Purine Binding Proteins

To test the concept of proteome mining of a combinatorial and natural products small molecule libraries for selective inhibitors of purine binding proteins, geldanamycin (GA) and 74 structural analogs were passed over gamma-phosphate linked ATP Sepharose that had previously been loaded with whole skeletal muscle extract. To ensure that all proteins that bind adenosine in the "protein kinase orientation" the resin contained an ATP concentration between 10-15 µMol/ml. Initial ligand screens were performed at 10 µM which would enable only pharmacologically relevant competitive inhibitors to be identified in the small library. This is because any protein that was selectively eluted from the ATP resin, by a particular GA analog, in order to have pharmacological relevance in subsequent cell based assays would have to be able to compete with a physiological ATP concentration of ~10 mM. As discussed earlier, the high ligand concentration also ensured that proteins of both high and low affinity, and copy number would be equally and maximally recovered by the resin. Fig. 3 shows a silver stain of peak column fractions after eluting the affinity resin with increasing concentrations of GA. A similar gel was transferred to PVM and the most abundant proteins identified by mixed peptide sequencing.

Washing the affinity column with 10 nM GA was found to almost exclusively elute a single protein at 45kDa. The fraction also contained a minor amount of a 90 kDa protein. Mixed peptide sequencing identified the 45kDa protein as ADE2 and the 90kDa protein as HSP90. In particular, mixed peptide sequencing identified peptide sequences Met Phe Phe Lys Asp Asp Ala Asn Asn Asp Pro Gln Trp (SEQ ID NO: 1) and Met Lys Ile Glu Phe Gly Val Asp Val Thr Thr Lys Glu (SEQ ID NO: 2) which were 100% identical to peptide sequences of purine multifunctional enzyme (ADE2). Mixed peptide sequencing also identified peptide sequences Met Thr Lys Ala Asp Leu Ile Asn Asn (SEQ ID NO: 3) and Met Ile Gly Gln Phe Gly Val Gly Phe Tyr (SEQ ID NO: 4) which were 100% identical to peptide sequences of heat shock protein 90 beta (HSP90).

These findings identify ADE2 as a new target for GA *in vitro.* PTH analysis of the sequenced proteins indicated that there were 2 pmols of ADE2 in the gel compared with <150 fmol of HSP90. Increasing the concentration of GA to 100 nM eluted 2 proteins of 70kDa and 65 kDa respectively that were identified by mixed peptide sequencing as proteolytic fragments of HSP90. No other proteins were eluted from the resin until the concentration of GA reached 10 µM. Approximately 1pmol of HSP90 was recovered in the gel at this step. Increasing the concentration of GA to 100 µM eluted a large amount of HSP90 and several N terminal proteolytic fragments of the protein. Approximately 11 pmols of the proteins was sequenced from the gel.

At 1mM GA 3 pmols of HSP90 was sequenced from the gel. Significantly no other proteins were eluted at this concentration. Subsequent, elution of the column with 10 mM ATP liberated a complex mixture of proteins of varying abundance and molecular weights. Consistent with previous results, mixed peptide sequencing of a selection of these proteins identified them as adenine nucleotide binding proteins.

The finding that the GA concentration could be raised by 3 orders magnitude over the concentration required to elute ADE2 before significant HSP90 was recovered demonstrates that in solution GA prefers the former enzyme over the later. Although, increasing the concentration to 1 mM did not elute any other proteins and is a testament to the selective of GA towards HSP90 and ADE2, these findings have implications for the actions of GA *in vivo.* In particular, the elution of ADE2 by GA illustrates a potential unforeseen toxicity of GA. The enzyme ADE2 catalyzes two essential steps that are required for the synthesis of purine nucleotides. Inhibition of ADE2 activity would therefore be toxic to all cell types.

To discriminate functional regions on GA that may discriminate sites of interaction with HSP90 from ADE2, 74 structural analogs (Table 2) of the molecule were passed over gamma-phosphate linked ATP Sepharose that had been charged with skeletal muscle extract.

Each analog of GA was washed over the gamma-phosphate linked ATP Sepharose that had been charged with skeletal muscle extract at 10 µM and the elutes analyzed by fluorography and SDSPAGE following tagging of the eluted proteins with iodofluorescein. The eluted proteins were visualized by laser induced fluorescence using a molecular dynamics flat bed imager. Using this method proteins that contained at least one reduced cysteine residue could be detected in the eluate at <0.1 fmol. Following fluorophor labeling the eluted proteins were characterized by SDSPAGE and fluorography. Mixed peptide sequencing identified the eluted proteins as either HSP90 or ADE2.
Several compounds within the small GA analog library have selectivity for HSP 90, while others are more selective for ADE2.

Assay of purified rabbit and human ADE2 confirmed that all compounds that selectively eluted ADE2 from the affinity also inhibited the enzyme in vitro (Table 2). All assays were performed against purified human ADE2. Results shown are from three separate experiments. Compounds that eluted HSP90 selectively had no effect of ADE2 activity in vitro at µM concentrations. Significantly, compounds that selectively eluted HSP90 showed low biological activity in cell based growth inhibition assays. In contrast, compounds that showed selectivity for ADE2 were potent inhibitors of cell growth. These findings demonstrate that *in vivo,* the biological effects of geldanamycin are because of its ability to inhibit ADE2 activity rather than through any actions on HSP90.

**Table 2. Determination of apparent Ki for ADE2 against GA structural analogs**

| **Inhibitor** | **Kᵢ⁻¹(µM)** | **Inhibitor** | **Kᵢ⁻¹(µM)** |
|---|---|---|---|
| 210760 | 6.803 | 683663 | 37.037 |
| 189794 | 3.546 | 330506 | 45.455 |
| 182857 | 5.525 | 255109 | 38.462 |
| 189795 | 0.688 | 320877 | 30.303 |
| 255111 | 5.051 | 665479 | 2.037 |
| 189793 | 3.497 | 265482 | 10.417 |
| 604169 | 38.462 | 320947 | 21.277 |
| 697886 | 15.152 | 182858 | 58.824 |
| 683662 | 16.667 | 156219 | 1000.000 |
| 672165 | 1.250 | 683660 | 90.909 |
| 330500 | 500.000 | 320946 | 33.333 |
| 661581 | 6.250 | 330509 | 21.277 |
| 255107 | 62.500 | 156217 | 22.222 |
| 683201 | 11.905 | 169627 | 7.634 |
| 655480 | 7.143 | 156218 | 58.824 |
| 255104 | 23.810 | 359658 | 1.815 |
| 682299 | 13.514 | 658515 | 0.484 |
| 655746 | 0.688 | 236651 | 34.483 |
| 682300 | 18.868 | 651937 | 0.185 |
| 330510 | 12.658 | 330499 | 0.615 |
| 683666 | 90.909 | 683664 | 1.692 |
| 661580 | 14.493 | 707545 | 62.500 |
| 662199 | 58.824 | 210753 | 0.362 |
| 690214 | 17.544 | 662199 | 50.000 |
| 607306 | 0.792 | 320944 | 20.000 |
| 607307 | 1.629 | 236652 | 10.526 |
| 255110 | 22.222 | 48810 | 14.706 |
| 321593 | 20.833 | 156216 | 26.316 |
| 674124 | 13.699 | 19990 | 20.408 |
| 156215 | 45.455 | 210761 | 27.778 |

## Claims

1. A method of identifying target compounds bioactive towards one or more proteins present in a proteome, which comprises:
providing a solid support on which a ligand is immobilised;
contacting the solid support with protein members of a proteome to bind some of said protein members to said ligand on said support;
washing the support to remove non-specifically bound proteins;
contacting the support with one or more target compounds that compete with said immobilised ligand for a binding site on one or more of said protein members of said proteome to release one or more bound proteome proteins;
collecting the protein or proteins released from said support by said one or more target compounds; and
determining for the target compound or compounds by sequencing or mass spectroscopy and comparison to protein databases the identity of the protein or proteins released from the support for identification of target compounds.

2. The method of claim 1, wherein the target compound is the same as the immobilized ligand.

3. The method of claim 1, wherein the target compound is a functional analog of the immobilized ligand.

4. The method of claim 1, wherein the target compound comprises a pharmaceutical.

5. The method of claim 4, wherein the pharmaceutical is a hormone, chemotherapeutic, nucleic acid, drug, antibiotic, antifungal or antiviral.

6. The method of claim 1, wherein the target compound comprises an insecticide or herbicide.

7. The method of any of claims 1 to 6, wherein the ligand is covalently bound to the solid support.

8. The method of any of claims 1 to 7, wherein the solid support comprises a cell or bacteriophage particle, an acrylamide derivative, sepharose, agarose, cellulose, nylon, silica, or magnetized particles.

9. The method of any of claims 1 to 8, wherein the proteome is derived from mammalian tissue, plant tissue or bacteria.

10. The method of claim 9, wherein the mammalian tissue is human tissue.

11. The method of any of claims 1 to 10, further comprising the step of labelling the bound proteins before the step of contacting the support with the target compound or compounds.

12. The method of any of claims 1 to 11, comprising the step of washing the support with the ligand and bound proteome on it with a buffered solution prior to contacting the support with one or more target compounds.

## Patentansprüche

1. Verfahren zum Identifizieren von Zielverbindungen, die gegenüber einem oder mehreren, in einem Proteom vorhandenen Proteinen biologisch aktiv sind, welches umfaßt:
Bereitstellen eines festen Trägers, auf dem ein Ligand immobilisiert ist;
in Kontakt bringen des festen Trägers mit Proteinbestandteilen eines Proteoms, so daß ein Teil der Proteinbestandteile an den Liganden auf dem Träger gebunden wird;
Waschen des Trägers, so daß nicht spezifisch gebundene Proteine entfernt werden;
in Kontakt bringen des Trägers mit einer oder mehreren Zielverbindungen, die mit dem immobilisierten Liganden für einen Bindungsort auf einem oder mehreren Proteinbestandteilen des Proteoms konkurrieren, so daß ein oder mehrere gebundene Proteom-Proteine freigesetzt werden;
Auffangen des Proteins oder der Proteine, das bzw. die durch die eine oder mehreren Zielverbindungen vom Träger freigesetzt wurde bzw. wurden; und
Bestimmen der Zielverbindung oder -verbindungen durch Sequenzananlyse oder Massenspektroskopie und Vergleichen der Identität des Proteins oder der Proteine, das bzw. die vom Träger freigesetzt wurde bzw. wurden, mit Protein-Datenbanken für eine Identifizierung von Zielverbindungen.

2. Verfahren nach Anspruch 1, wobei die Zielverbindung die gleiche wie der immobilisierte Ligand ist.

3. Verfahren nach Anspruch 1, wobei die Zielverbindung ein funktionelles Analogon des immobilisierten Liganden ist.

4. Verfahren nach Anspruch 1, wobei die Zielverbindung ein Arzneimittel umfaßt.

5. Verfahren nach Anspruch 4, wobei das Arzneimittel ein Hormon, chemotherapeutisch, eine Nucleinsäure, ein Wirkstoff, antibiotisch, antifungizid oder antiviral ist.

6. Verfahren nach Anspruch 1, wobei die Zielverbindung ein Insektizid oder Herbizid umfaßt.

7. Verfahren nach einem der Ansprüche 1 bis 6, wobei der Ligand kovalent an den festen Träger gebunden ist.

8. Verfahren nach einem der Ansprüche 1 bis 7, wobei der feste Träger eine Zelle oder ein Bakteriophagenpartikel, ein Acrylamid-Derivat, Sepharose, Agarose, Cellulose, Nylon, Kieselerde oder magnetisierte Partikel umfaßt.

9. Verfahren nach einem der Ansprüche 1 bis 8, wobei das Proteon von Mammaliagewebe, Pflanzengewebe oder Bakterien stammt.

10. Verfahren nach Anspruch 9, wobei das Mammaliagewebe Humangewebe ist.

11. Verfahren nach einem der Ansprüche 1 bis 10, das vor dem Schritt des Kontaktes des Trägers mit der Zielverbindung oder den -verbindungen ferner den Schritt des Markierens der gebundenen Proteine umfaßt.

12. Verfahren nach einem der Ansprüche 1 bis 11, das vor dem Kontakt des Trägers mit einer oder mehreren Zielverbindungen den Schritt des Waschens des Trägers mit dem Liganden und dem darauf gebundenen Proteom mit einer gepufferten Lösung umfaßt.

## Revendications

1. Procédé d'identification de composés cibles bioactifs envers une ou plusieurs protéines présentes dans un protéome, qui comprend les étapes consistant à :
- fournir un support solide sur lequel un ligand est immobilisé ;
- mettre en contact le support solide avec des éléments protéiques d'un protéome pour lier une partie des éléments protéiques audit ligand sur ledit support ;
- laver le support pour éliminer les protéines liées de manière non spécifique ;
- mettre en contact le support avec un ou plusieurs composés cibles qui entrent en compétition avec ledit ligand immobilisé pour un site de liaison sur un ou plusieurs desdits éléments protéiques dudit protéome pour libérer une ou plusieurs protéines du protéome liées ;
- recueillir la ou les protéines libérées dudit support par lesdits un ou plusieurs composés cibles ; et
- déterminer le ou les composés cibles par séquençage ou spectroscopie de masse et comparaison avec des bases de données de protéines, de l'identité de la ou des protéines libérées du support pour l'identification de composés cibles.

2. Procédé selon la revendication 1, dans lequel le composé cible est le même que le ligand immobilisé.

3. Procédé selon la revendication 1, dans lequel le composé cible est un analogue fonctionnel du ligand immobilisé.

4. Procédé selon la revendication 1, dans lequel le composé.cible comprend un agent pharmaceutique.

5. Procédé selon la revendication 4, dans lequel l'agent pharmaceutique est une hormone, un agent chimiothérapeutique, un acide nucléique, un médicament, un antibiotique, un agent antifongique ou antiviral.

6. Procédé selon la revendication 1, dans lequel le composé cible comprend un insecticide ou un herbicide.

7. Procédé selon l'une quelconque des revendications 1 à 6, dans lequel le ligand est lié par liaison covalente au support solide.

8. Procédé selon l'une quelconque des revendications 1 à 7, dans lequel le support solide comprend une cellule ou une particule de bactériophage, un dérivé d'acrylamide, du Sepharose, de l'agarose, de la cellulose, du nylon, de la silice ou des particules aimantées.

9. Procédé selon l'une quelconque des revendications 1 à 8, dans lequel le protéome est dérivé d'un tissu mammifère, d'un tissu végétal ou d'une bactérie.

10. Procédé selon la revendication 9, dans lequel le tissu mammifère est un tissu humain.

11. Procédé selon l'une quelconque des revendications 1 à 10, comprenant en outre l'étape consistant à marquer les protéines liées, avant l'étape de mise en contact du support avec le ou les composés cibles.

12. Procédé selon l'une quelconque des revendications 1 à 11, comprenant l'étape consistant à laver le support avec le ligand et le protéome lié sur celui-ci, avec une solution tamponnée, avant la mise en contact du support avec un ou plusieurs composés cibles.


### SEQUENCE LISTING

<110> Haystead, Timothy A
   University of Virginia Patent Foundation
<120> Proteome Mining
<130> 00164-02
<140>
   <141>
<160> 4
<170> PatentIn Ver. 2.0
<210> 1
   <211> 13
   <212> PRT
   <213> Oryctolagus cuniculus
<400> 1
   Met Phe Phe Lys Asp Asp Ala Asn Asn Asp Pro Gln Trp
    1               5                  10
<210> 2
   <211> 13
   <212> PRT
   <213> Oryctolagus cuniculus
<400> 2
   Met Lys Ile Glu Phe Gly Val Asp Val Thr Thr Lys Glu
   1 5 10
<210> 3
   <211> 9
   <212> PRT
   <213> Oryctolagus cuniculus
<400> 3
   Met Thr Lys Ala Asp Leu Ile Asn Asn
   1 5
<210> 4
   <211> 10
   <212> PRT
   <213> Oryctolagus cuniculus
<400> 4
   Met Ile Gly Gln Phe Gly Val Gly Phe Tyr
   1 5 10